# EUROPEAN PATENT SPECIFICATION

(11) **EP 3 893 732 B1**
(45) Date of publication and mention of the grant of the patent: **03.07.2024**
(21) Application number: 19839255.7
(22) Date of filing: 13.12.2019
(51) Int. Cl.: A61B 5/00

(54) **DEVICE FOR USE IN DETERMINING A HYDRATION LEVEL OF SKIN**
VORRICHTUNG ZUR VERWENDUNG BEI DER BESTIMMUNG DES HYDRATISIERUNGSGRADES DER HAUT
DISPOSITIF DESTINÉ À ÊTRE UTILISÉ DANS LA DÉTERMINATION D'UN NIVEAU D'HYDRATATION DE LA PEAU

(30) Priority: 14.12.2018 EP 18212723
(43) Date of publication of application: 20.10.2021
(73) Proprietor: Koninklijke Philips N.V., 5656 AG Eindhoven (NL)
(72) Inventor: VARGHESE, Babu, 5656 AE Eindhoven (NL); EZERSKAIA, Anna, 5656 AE Eindhoven (NL)
(74) Representative: Philips Intellectual Property & Standards
(86) International application number: PCT/EP2019/085198
(87) International publication number: WO 2020/120781

(56) References cited:
- EP-A1- 3 070 457
- US-A1- 2015 223 749
- US-A1- 2015 374 276

## Description

### FIELD OF THE INVENTION

The disclosure relates to a system for determining a hydration level of skin and a method of operating the system.

### BACKGROUND OF THE INVENTION

The hydration (e.g. moisture or water) level of skin is considered an important factor in determining skin appearance and skin health. The most widely used technique to measure the hydration level of skin is based on measuring the electrical properties of the skin, such as bio-impedance, capacitance, and conductance. However, these are indirect measurements and the results of these measurement techniques are confounded by several factors. Some of these factors include, for example, other polar molecules in the skin, skin temperature, contact pressure, and contact area. Furthermore, the most widely used skin hydration measurement devices (such as the Comeometer and Skicon) use inter-digitized electrodes, provide skin hydration information averaged over an area, and therefore do not provide information about the spatial variation in moisture content. More recently developed devices (such as the Epsilon and MoistureMap MM 100) use capacitive fingerprint sensing technology for providing information on the near surface skin hydration distribution.

Other existing techniques for estimating skin hydration include spectroscopic methods, which estimate skin hydration based on prominent water absorption bands and allows for a more direct measurement. It has been found that skin hydration measurements using the strongest water absorption bands are sufficient for the detection of changes in skin hydration content. However, the existing techniques that make use of this are expensive and there is also only very limited availability of the optical components required for the sensor used to detect the changes in skin hydration. Thus, optical systems utilizing wavelengths below the sensitivity of silicon material are generally more attractive due to their low-cost.

US 2011/0288385 discloses an example of a system comprising a sensor that utilizes lower wavelengths. However, the system cost and complexity is greater compared to the other techniques and the system does not necessarily provide accurate results for the measurement of skin hydration as the probing depth does not relate to the optimum stratum corneum layer thickness.

It is to be noted that US patent application publication 2015/0223749 A1 proposes a skin condition evaluation method in which a light source including one or more LEDs irradiates light onto skin and a light detector receives light emitted through the skin and an amount of a component distributed in the skin is derived from the received light.

It is further to be noted that US patent application publication 2015/0374276 A1 discloses a system for characterizing a portion of biological tissue by illuminating the tissue with light having a known wavelength spectrum capable of materially penetrating the tissue. The system is disclosed to measure the intensity of the illumination light remitted from the tissue in response to the illumination over a known measurement window over a hyperspectral range of wavelengths for at least two distinguishable polarization components.

It is further to be noted that European patent application publication 3 070 457 A1 discloses a device having an illumination unit that casts light of a prescribed polarization direction on a scattering body, a camera that picks up images of the scattering body at a plurality of different polarization angles, and a processor arranged to generate and output an inner layer image of an inner layer of an inside of the scattering body.

### SUMMARY OF THE INVENTION

As noted above, a limitation with existing systems is that the depth at which the skin can be probed does not relate to the optimum stratum corneum layer thickness and thus measurements of skin hydration obtained by the existing systems can be inaccurate. It would thus be valuable to have an improvement to address this limitation.

Therefore, according to a first aspect, there is provided system for determining a hydration level of skin. The system comprises a device. The device comprises a light source configured to emit light to illuminate skin and a first polarizer arranged to polarize light emitted from the light source in a first polarization direction to illuminate the skin with polarized light. The device also comprises a second polarizer arranged to polarize light from the skin in a second polarization direction. The second polarization direction is orientated at an angle in a range from 30 to 60 degrees with respect to the first polarization direction. The device also comprises a light detector configured to detect the polarized light from the skin for use in determining a hydration level of the skin. The light detector is configured to convert the detected polarized light from the skin into an image of the skin in a green channel. The system further comprises a processor configured to determine the hydration level of the skin based on the detected polarized light from the skin.

In some embodiments, the second polarization direction may be orientated at an angle in a range from 40 to 50 degrees with respect to the first polarization direction. In some embodiments, the second polarization direction may be orientated at an angle of 45 degrees with respect to the first polarization direction.

In some embodiments, the light source may be configured to emit near infrared light. In some embodiments, the light source may be configured to emit near infrared light with a wavelength in a range from 750 to 1550 nm. In some embodiments, the light source may be configured to emit near infrared light with a wavelength in a range from 950 to 1000 nm, 1150 to 1250 nm or 1400 to 1500 nm. In some embodiments, the light source may comprise a plurality of light emitters. In some embodiments, the light source may comprise at least three light emitters. In some embodiments, the plurality of light emitters may be configured to sequentially emit light. In some embodiments, the light source may be configured to emit light with an angle of incidence in a range from 10 to 70 degrees. In some embodiments, the light source may be configured to emit light with an angle of incidence of 22 degrees.

According to a second aspect, there is provided a method of operating the system as described earlier. The method comprises emitting the light from the light source, polarizing the emitted light in the first polarization direction, polarizing the light from the skin in the second polarization direction and detecting the polarized light from the skin for use in determining the hydration level of the skin.

According to a third aspect, there is provided a computer program product comprising a computer readable medium. The computer readable medium has computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, provide optimal illumination conditions for use in determining a hydration level of skin. In particular, having the second polarization direction orientated at an angle in a range from 30 to 60 degrees with respect to the first polarization direction provides a device that can probe the skin at a depth that is optimum for probing the stratum corneum. In particular, as the hydration increases as a function of depth within the thickness of the stratum corneum, having the second polarization direction orientated at an angle in a range from 30 to 60 degrees with respect to the first polarization direction means that the device can probe the skin at a depth that provides a more accurate representation of the skin hydration level of the stratum corneum. In this way, a more accurate determination of the hydration level of the skin can be provided. There is thus provided an improved device, system and method for use in determining a hydration level of skin.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified schematic illustration of a side view of a device according to an embodiment;
Fig. 2 is a simplified schematic illustration of a top view of a device according to an embodiment;
Fig. 3 is an illustration of an absorption coefficient of skin as a function of wavelength of light emitted to illuminate the skin;
Figs. 4(a) and (b) are illustrations of intensity as a function of skin hydration; and
Fig. 5 is a flow chart illustrating a method according to an embodiment.

### DETAILED DESCRIPTION OF THE EMBODIMENTS

As noted above, there is provided herein an improved device, system and method for use in determining a hydration (e.g. moisture or water) level of skin. The skin referred to herein can comprise one or more layers of skin, which can include the stratum corneum.

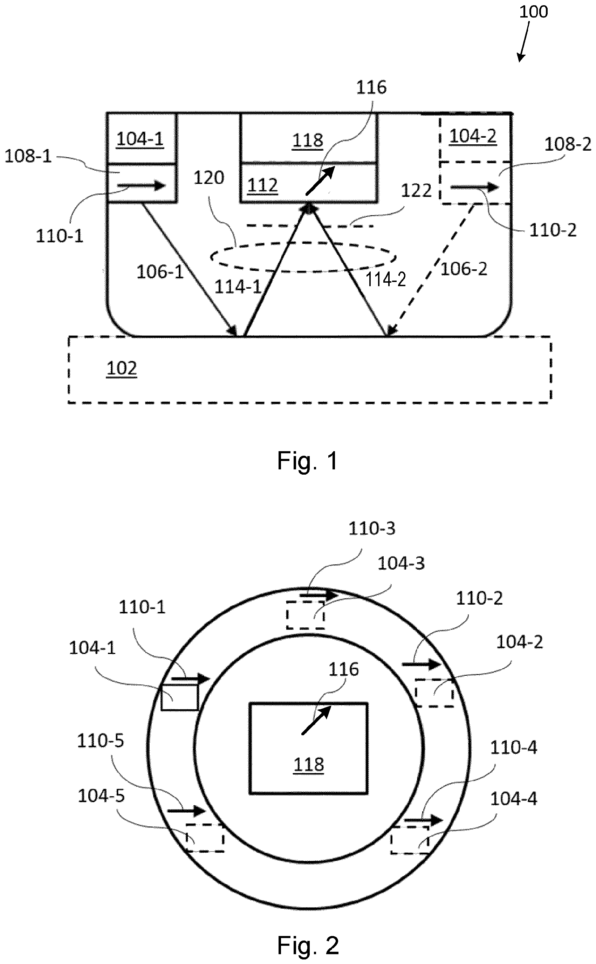

Fig. 1 illustrates a side view of a device 100 for use in determining a hydration level of skin according to an embodiment. As illustrated in Fig. 1, the device 100 comprises a light source 104-1, 104-2. In some embodiments, the light source 104-1, 104-2 can comprise one or more light emitting diodes (LED) and/or any other light source.

In some embodiments, the light source may comprise a single light emitter 104-1. In other embodiments, the light source may comprise a plurality of light emitters 104-1, 104-2. Although Fig. 1 illustrates that the light source may comprise one light emitter 104-1 or two light emitters 104-1, 104-2, it will be understood that the light source may comprise any other number of light emitters. For example, in some embodiments, the light source may comprise at least three light emitters, for example at least four light emitters, for example at least five light emitters, or any other number of light emitters. As illustrated in Fig. 1, the light source 104-1, 104-2 of the device 100 is configured to emit light 106-1, 106-2 to illuminate skin 102.

As also illustrated in Fig. 1, the device 100 comprises a first polarizer 108-1, 108-2. The first polarizer 108-1, 108-2 is arranged to polarize light 106-1, 106-2 emitted from the light source 104-1, 104-2 in a first polarization direction 110-1, 110-2 to illuminate the skin 102 with polarized light 106-1, 106-2. In embodiments where the light source 104-1, 104-2 comprises a plurality of light emitters, the device 100 can comprise a respective plurality of first polarizers 108-1, 108-2. For each of the a plurality of light emitters 104-1, 104-2, the respective first polarizer 108-1, 108-2 can be arranged to polarize light 106-1, 106-2 emitted from that light emitter 104-1, 104-2 in the first polarization direction 110-1, 110-2 to illuminate the skin 102 with polarized light 106-1, 106-2.

In some embodiments, a first medium that the polarized light 106-1, 106-2 passes through in the device 100 before reaching the skin 102 can comprise air. In some embodiments, a second medium that the polarized light 106-1, 106-2 after leaving the device 100 is the skin 102, e.g. the stratum corneum of the skin 102. When the polarized light 106-1, 106-2 illuminates the skin 102, a percentage (e.g. approximately 4%) of the polarized light 106-1, 106-2 incident on the skin 102 may be specularly reflected based on Fresnel's law. The rest of the polarized light 106-1, 106-2 incident on the skin 102 penetrates the skin 102 (or, more specifically, the skin tissue). Once the polarized light 106-1, 106-2 penetrates the skin 102, the polarized light 106-1, 106-2 may then undergo scattering and/or reflection events in the skin 102. The polarized light 106-1, 106-2 either exits the skin 102 after a few scattering events in the stratum corneum of the skin 102 or penetrates deeper in the skin 102 in which case the polarized light 106-1, 106-2 undergoes multiple reflection events before exiting the skin 102. The polarized light 106-1, 106-2 that exits the skin 102 after a few scattering events in the stratum corneum of the skin 102 is specular light, which maintains its polarization state when exiting the skin 102. The polarized light 106-1, 106-2 that undergoes multiple reflection events before exiting the skin 102 is diffuse light, which does not maintain its polarization state when exiting the skin 102.

As illustrated in Fig. 1, the device 100 also comprises a second polarizer 112. The second polarizer 112 is arranged to polarize light 114-1, 114-2 from the skin 102 in a second polarization direction 116. The second polarization direction 116 is orientated at an angle in a range from 30 to 60 degrees with respect to the first polarization direction 110-1, 110-2. For example, in some embodiments, the second polarization direction 116 may be orientated at an angle in a range from 35 to 55 degrees with respect to the first polarization direction 110-1, 110-2, for example in a range from 40 to 50 degrees with respect to the first polarization direction 110-1, 110-2. For example, in some embodiments, the second polarization direction 116 may be orientated at an angle with respect to the first polarization direction 110-1, 110-2, which is an angle selected from 30 degrees, 35 degrees, 40 degrees, 45 degrees, 50 degrees, 55 degrees, 60 degrees, or any other integer or non-integer angle between 30 degrees and 60 degrees.

As illustrated in Fig. 1, the device 100 also comprises a light detector (or imaging sensor) 118. The light detector 118 is configured to detect the polarized light 114-1, 114-2 from the skin 102 for use in determining a hydration level of the skin. As the second polarization direction 116 is orientated at an angle in a range from 30 to 60 degrees with respect to the first polarization direction 110-1, 110-2, it can be ensured that both specular and diffuse light is detected from the skin 102 for use in determining a hydration level of the skin. By having the first and second polarization 110-1, 110-2, 116 directions orientated with respect to each other in this particular way means that the device 100 can probe the skin 102 at a depth that is optimum for probing the stratum corneum. In particular, as the hydration increases as a function of depth within the thickness of the stratum corneum, having the second polarization direction 116 orientated at an angle in a range from 30 to 60 degrees with respect to the first polarization direction 110-1, 110-2 means that the device 100 can probe the skin 102 at a depth that provides a more accurate representation of the skin hydration level of the stratum corneum. In this way, a more accurate determination of the hydration level of the skin 102 can be provided.

In some embodiments, the light detector 118 may comprise a camera (e.g. a color camera) or any other light detector. In some embodiments, the light detector 118 may have a fixed focus. In some embodiments, the light detector 118 may comprise a plurality of pixels. The plurality of pixels may be selected so as to reduce processing overhead. As an example, the light detector 118 may comprise 2592 × 1944 pixels. In some embodiments, as illustrated in Fig. 1, the device 100 may comprise a lens 120. The lens 120 can be arranged (e.g. positioned) between the light detector 118 and the skin 102. More specifically, the lens 120 can be arranged (e.g. positioned) between the light detector 118 and the skin 102 such that the light 114-1, 114-2 from the skin 102 passes through the lens 120 before reaching the light detector 118. The lens can be configured to set a field of view for the light detector 118 and/or to focus the light 114-1, 114-2 from the skin 102 on the light detector 118. For example, a lens 120 having a focal length f of 10 mm may be used to achieve a field of view of approximately 10 × 7.5 mm and a focus at approximately 10 mm.

In some embodiments, as illustrated in Fig. 1, the device 100 may comprise a structure (e.g. a diaphragm) 122 having an aperture. The structure 122 having the aperture can be arranged (e.g. positioned) between the light detector 118 and the skin 102. More specifically, the structure 122 having the aperture can be arranged (e.g. positioned) between the light detector 118 and the skin 102 such that the light 114-1, 114-2 from the skin 102 passes through the structure 122 before reaching the light detector 118. In some embodiments, the aperture of the structure 122 may have a diameter in a range from 0.6 to 1.16 mm. A larger depth of focus may be achieved with a smaller diameter, e.g. of 0.6 mm. For example, a depth of focus may be 611 µm. In some embodiments, a resolving power of the light detector 118 may be 10 µm.

Fig. 2 illustrates a top view of a device 100 for use in determining a hydration level of skin 102 according to an embodiment. The device 100 illustrated in Fig. 2 is as described earlier with reference to Fig. 1. As illustrated in Fig. 2, in some embodiments where the light source comprises a plurality of light emitters 104-1, 104-2, 104,-3, 104-4, 104-5, the plurality of light emitters 104-1, 104-2, 104,-3, 104-4, 104-5 may be arranged (e.g. positioned) in a spatial arrangement with respect to the light detector 118, such as by being arranged (e.g. positioned) around the light detector 118. In some embodiments, for example, as illustrated in Fig. 2, the plurality of light emitters 104-1, 104-2, 104,-3, 104-4, 104-5 may be arranged (e.g. positioned) concentrically around the light detector 118. Although Fig. 2 illustrates that the light source may comprise one light emitter 104-1, two light emitters 104-1, 104-2, three light emitters 104-1, 104-2, 104-3, four light emitters 104-1, 104-2, 104-3, 104-4, or five light emitters 104-1, 104-2, 104-3, 104-4, 104-5, it will be understood that the light source may comprise any other number of light emitters as described earlier.

As illustrated in Fig. 2 and as described earlier with reference to Fig. 1, the first polarizer of the device 100 (not depicted in Fig. 2) is arranged to polarize light (not depicted in Fig. 2) emitted from the light source 104-1, 104-2, 104,-3, 104-4, 104-5 in a first polarization direction 110-1, 110-2, 110-3, 110-4, 110-5 to illuminate the skin (not depicted in Fig. 2) with polarized light. The second polarizer (not depicted in Fig. 2) is arranged to polarize light from the skin (not depicted in Fig. 2) in a second polarization direction 116. As described earlier with reference to Fig. 1, the second polarization direction 116 is orientated at an angle in a range from 30 to 60 degrees with respect to the first polarization direction 110-1, 110-2, 110-3, 110-4, 110-5. For example, in some embodiments, the second polarization direction 116 may be orientated at an angle in a range from 35 to 55 degrees with respect to the first polarization direction 110-1, 110-2, 110-3, 110-4, 110-5, for example in a range from 40 to 50 degrees with respect to the first polarization direction 110-1, 110-2, 110-3, 110-4, 110-5. For example, in some embodiments, the second polarization direction 116 may be orientated at an angle with respect to the first polarization direction 110-1, 110-2, 110-3, 110-4, 110-5, which is an angle selected from 30 degrees, 35 degrees, 40 degrees, 45 degrees, 50 degrees, 55 degrees, 60 degrees, or any other integer or non-integer angle between 30 degrees and 60 degrees.

In some embodiments, the light source 104-1, 104-2, 104,-3, 104-4, 104-5 of the device 100 described herein with reference to Figs. 1 and 2 may be configured to emit near infrared light. For example, in some embodiments, the light source 104-1, 104-2, 104,-3, 104-4, 104-5 of the device 100 described herein with reference to Figs. 1 and 2 may be configured to emit near infrared light with a wavelength in a range from 750 to 1550 nm. In some embodiments, the light source 104-1, 104-2, 104,-3, 104-4, 104-5 of the device 100 described herein with reference to Figs. 1 and 2 may be configured to emit near infrared light with a wavelength at which a prominent water absorption peak occurs.

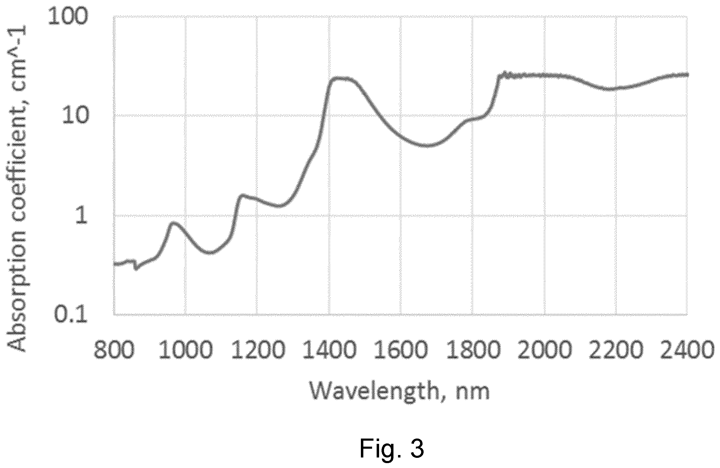

Fig. 3 illustrates an absorption coefficient of skin 102 as a function of wavelength of light emitted to illuminate the skin 102. As illustrated in Fig. 3, a prominent water absorption peak occurs in a range from 950 to 1000 nm, in a range from 1150 to 1250 nm, and in a range from 1400 to 1500 nm. Thus, in some embodiments, the light source 104-1, 104-2, 104,-3, 104-4, 104-5 of the device 100 described herein with reference to Figs. 1 and 2 may be configured to emit near infrared light with a wavelength in a range from 950 to 1000 nm, for example a wavelength in a range from 960 to 990 nm, for example a wavelength in a range from 970 to 980 nm. For example, in some embodiments, the light source 104-1, 104-2, 104,-3, 104-4, 104-5 of the device 100 described herein with reference to Figs. 1 and 2 may be configured to emit near infrared light with a wavelength selected from 950 nm, 960 nm, 970 nm, 980 nm, 990 nm, 1000 nm, or any other integer or non-integer wavelength between 950 nm and 1000 nm.

In some embodiments, the light source 104-1, 104-2, 104,-3, 104-4, 104-5 of the device 100 described herein with reference to Figs. 1 and 2 may be configured to emit near infrared light with a wavelength in a range from 1150 to 1250 nm, for example a wavelength in a range from 1160 to 1240 nm, for example a wavelength in a range from 1170 to 1230 nm, for example a wavelength in a range from 1180 to 1220 nm, for example a wavelength in a range from 1190 to 1210 nm. For example, in some embodiments, the light source 104-1, 104-2, 104,-3, 104-4, 104-5 of the device 100 described herein with reference to Figs. 1 and 2 may be configured to emit near infrared light with a wavelength selected from 1150 nm, 1160 nm, 1170 nm, 1180 nm, 1190 nm, 1200 nm, 1210 nm, 1220 nm, 1230 nm, 1240 nm, 1250 nm, or any other integer or non-integer wavelength between 1150 nm and 1250 nm.

In some embodiments, the light source 104-1, 104-2, 104,-3, 104-4, 104-5 of the device 100 described herein with reference to Figs. 1 and 2 may be configured to emit near infrared light with a wavelength in a range from 1400 to 1500 nm, for example a wavelength in a range from 1410 to 1490 nm, for example a wavelength in a range from 1420 to 1480 nm, for example a wavelength in a range from 1430 to 1470 nm, for example a wavelength in a range from 1440 to 1460 nm. For example, in some embodiments, the light source 104-1, 104-2, 104,-3, 104-4, 104-5 of the device 100 described herein with reference to Figs. 1 and 2 may be configured to emit near infrared light with a wavelength selected from 1400 nm, 1410 nm, 1420 nm, 1430 nm, 1440 nm, 1450 nm, 1460 nm, 1470 nm, 1480 nm, 1490 nm, 1500 nm, or any other integer or non-integer wavelength between 1400 nm and 1500 nm.

In some embodiments where the light source of the device 100 described herein with reference to Figs. 1 and 2 comprises a plurality of light emitters 104-1, 104-2, 104,-3, 104-4, 104-5, the plurality of light emitters 104-1, 104-2, 104,-3, 104-4, 104-5 can be configured to sequentially emit light. In some embodiments, the light source of the device 100 described herein with reference to Figs. 1 and 2 may be configured to emit light with an angle of incidence in a range from 10 to 70 degrees, for example in a range from 15 to 65 degrees, for example in a range from 20 to 60 degrees, for example in a range from 25 to 55 degrees, for example in a range from 30 to 50 degrees, for example in a range from 35 to 45 degrees. For example, in some embodiments, the light source of the device 100 described herein with reference to Figs. 1 and 2 may be configured to emit light with an angle of incidence selected from 10 degrees, 12 degrees, 14 degrees, 16 degrees, 18 degrees, 20 degrees, 22 degrees, 24 degrees, 26 degrees, 28 degrees, 30 degrees, 32 degrees, 34 degrees, 36 degrees, 38 degrees, 40 degrees, 42 degrees, 44 degrees, 46 degrees, 48 degrees, 50 degrees, 52 degrees, 54 degrees, 56 degrees, 58 degrees, 60 degrees, 62 degrees, 64 degrees, 66 degrees, 68 degrees, 70 degrees, or any other integer or non-integer angle of incidence between 10 and 70 degrees.

The light detector 118 of the device 100 described herein with reference to Figs. 1 and 2 is configured to convert the detected polarized light 114-1, 114-2 from the skin 102 into an image of the skin 102. In some embodiments, the light detector 118 of the device 100 described herein with reference to Figs. 1 and 2 may be configured to convert the detected polarized light 114-1, 114-2 from the skin 102 into the image of the skin 102 in any one or more of a red channel, a blue channel, and a green channel. The present invention is limited to embodiments in which the green channel is used to determine a hydration level of the skin. In some embodiments, the light detector 118 of the device 100 described herein with reference to Figs. 1 and 2 may be configured to apply flat field correction (FFC) to the image of the skin 102. In other embodiments, the light detector 118 of the device 100 described herein with reference to Figs. 1 and 2 may be configured to convert the detected polarized light 114-1, 114-2 from the skin 102 into an image of the skin 102 without applying FFC to the image.

Although not illustrated in the figures, there is also provided a system comprising the device 100 as described earlier with reference to Figs. 1 and 2. In some embodiments, the system may also comprise a processor. The processor can be implemented in numerous ways, with software and/or hardware, to perform the various functions described herein. In particular implementations, the processor can comprise a plurality of software and/or hardware modules, each configured to perform, or that are for performing, individual or multiple steps of the method described herein. The processor may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The processor may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

The processor is configured to determine the hydration level of the skin 102 based on the detected polarized light 114-1, 114-2 from the skin 102. For example, the hydration level of the skin 102 may be determined as the average intensity of the detected polarized light 114-1, 114-2 from the skin 102. In some embodiments, the average intensity of the detected polarized light 114-1, 114-2 from the skin 102 may be determined from the image of the skin 102. For example, the average intensity of the detected polarized light 114-1, 114-2 from the skin 102 may be determined as the average of the sum of the pixels in the image of the skin 102. In some embodiments, only the detected polarized light 114-1, 114-2 that corresponds to the diffuse light from the skin 102 may be used in the determination of the average intensity. In some embodiments, this average intensity of the detected polarized light 114-1, 114-2 that corresponds to the diffuse light from the skin 102 may be corrected using a factor derived from a determined average intensity of the detected polarized light 114-1, 114-2 that corresponds to the specular light from the skin 102.

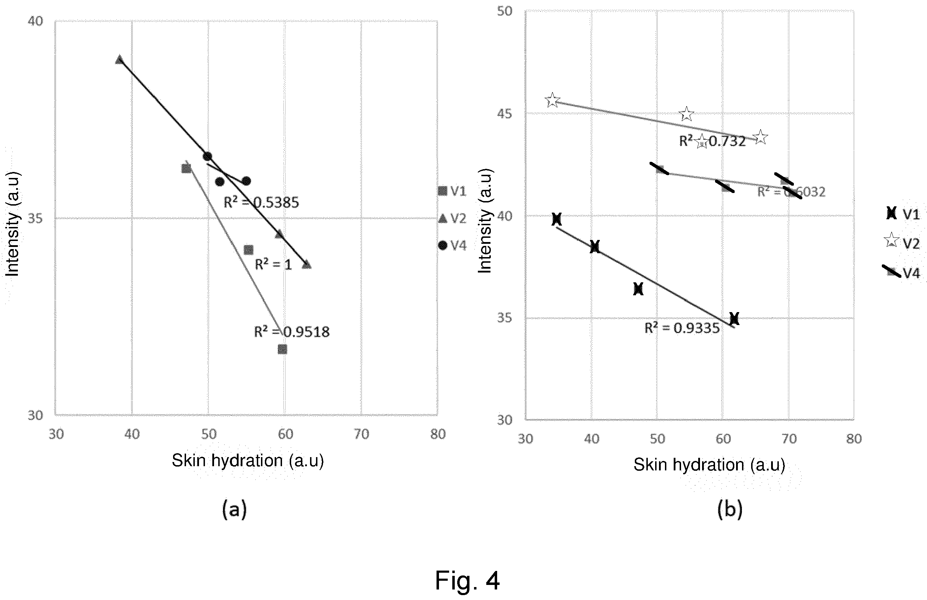

Figs. 4(a) and (b) illustrate an average intensity of polarized light 114-1, 114-2 detected from skin 102 by the light detector 118 of the device 100 described herein as a function of skin hydration measurements obtained with a reference device for three subjects V1, V2, V4. More specifically, Figs. 4(a) and (b) illustrate the average intensity of polarized light 114-1, 114-2 detected from skin 102 by the light detector 118 of the device 100 described herein as a function of skin hydration measurements obtained with a reference device for the three subjects V 1, V2, V4 under natural conditions and under forced hydration conditions respectively. The reference device is a Comeometer. The settings of the device 100 described herein that were used to detect the polarized light 114-1, 114-2 included the second polarization direction 116 being orientated at an angle of 45 degrees with respect to the first polarization direction 110-1, 110-2, 110-3, 110-4, 110-5, the light source comprising five light emitters 104-1, 104-2, 104,-3, 104-4, 104-5 configured to sequentially emit light to illuminate the skin 102 at a wavelength of 970 nm, and the light emitters being LEDs.

As illustrated by the coefficient of determination (R²) values in Figs. 4(a) and (b), the results show a good correlation between the skin hydration measurements obtained with the reference device and the average intensity of polarized light 114-1, 114-2 detected from skin 102 by the light detector 118 of the device 100 described herein.

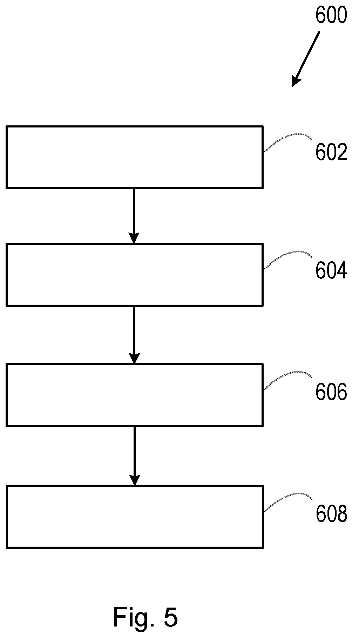

Fig. 5 is a flow chart illustrating a method 600 according to an embodiment. More specifically, the method 600 of Fig. 5 is a method of operating the device 100 described earlier with reference to Figs. 1 and 2. The method 600 may generally be performed by or under the control of a processor, such as that described earlier.

With reference to Fig. 5, at block 602, the light 106-1, 106-2 is emitted from the light source 104-1, 104-2, 104,-3, 104-4, 104-5. For example, in some embodiments, the processor may control the light source104-1, 104-2, 104,-3, 104-4, 104-5 to emit the light 106-1, 106-2. At block 604 of Fig. 5, the emitted light 106-1, 106-2 is polarized in the first polarization direction 110-1, 110-2, 110-3, 110-4, 110-5. More specifically, the first polarizer 108-1, 108-2 polarizes light 106-1, 106-2 emitted from the light source 104-1, 104-2, 104,-3, 104-4, 104-5 in the first polarization direction 110-1, 110-2, 110-3, 110-4, 110-5 to illuminate the skin 102 with polarized light 106-1, 106-2.

At block 606 of Fig. 5, the light 114-1, 114-2 from the skin 102 is polarized in the second polarization direction 116. More specifically, the second polarizer 112 polarizes light 114-1, 114-2 from the skin 102 in the second polarization direction 116. As described earlier, the second polarization direction 116 is orientated at an angle in a range from 30 to 60 degrees with respect to the first polarization direction 110-1, 110-2, 110-3, 110-4, 110-5. At block 608 of Fig. 5, the polarized light from the skin is detected for use in determining the hydration level of the skin. More specifically, the light detector 118 detects the polarized light 114-1, 114-2 from the skin 102 for use in determining a hydration level of the skin 102. For example, in some embodiments, the processor may control the light detector 118 to detect the polarized light 114-1, 114-2 from the skin 102 for use in determining a hydration level of the skin 102.

The polarized light 114-1, 114-2 from the skin 102 detected by the light detector 118 of the device described herein can provide spatial information on skin hydration, rather than point measurements. For example, in some embodiments, the polarized light 114-1, 114-2 from the skin 102 detected by the light detector 118 of the device described herein can be used in measuring a hydration (e.g. moisture or water) profile or map of the skin 102. The profile or map can be a digital profile or map of the skin 102. This can be useful for users to visualize and quantify the hydration level. The device 100 described herein can be easily integrated into current face smart sensor platforms without the need for complex system upgrades.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein a device 100, a system, a method 600 and a computer program product that address the limitations associated with the existing techniques. Therefore, there is provided an improved device 100, a system, a method 600 and a computer program product for use in determining a hydration level of skin 102. The hydration level of skin 102 can be used to determine (or assess) skin appearance and skin health. The hydration level of skin 102 may also be used to classify different hydrating products according to their moisturizing effect induced on the skin, monitor changes in molecular concentration or concentration profiles induced by topical application, and identify other types of hydrating agents.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for determining a hydration level of skin (102), wherein the system comprises a device (100), the device (100) comprising:
a light source (104-1, 104-2, 104,-3, 104-4, 104-5) configured to emit light (106-1, 106-2) to illuminate skin (102);
a first polarizer (108-1, 108-2) arranged to polarize light (106-1, 106-2) emitted from the light source (104-1, 104-2, 104,-3, 104-4, 104-5) in a first polarization direction (110-1, 110-2, 110-3, 110-4, 110-5) to illuminate the skin (102) with polarized light (106-1, 106-2);
a second polarizer (112) arranged to polarize light (114-1, 114-2) from the skin (102) in a second polarization direction (116), wherein the second polarization direction (116) is orientated at an angle in a range from 30 to 60 degrees with respect to the first polarization direction (110-1, 110-2, 110-3, 110-4, 110-5); and
a light detector (118) configured to detect the polarized light (114-1, 114-2) from the skin (102);
wherein the light detector (118) is configured to convert the detected polarized light (114-1, 114-2) from the skin (102) into an image of the skin (102) in a green channel;
wherein the system comprises a processor configured to:
determine a hydration level of the skin (102) based on the detected polarized light (114-1, 114-2) in the image of the skin (102) in a green channel.

2. The system as claimed in claim 1, wherein the second polarization direction (116) is orientated at an angle in a range from 40 to 50 degrees with respect to the first polarization direction (110-1, 110-2, 110-3, 110-4, 110-5).

3. The system as claimed in claim 2, wherein the second polarization direction (116) is orientated at an angle of 45 degrees with respect to the first polarization direction (110-1, 110-2, 110-3, 110-4, 110-5).

4. The system as claimed in any of the preceding claims, wherein the light source (104-1, 104-2, 104,-3, 104-4, 104-5) is configured to emit near infrared light.

5. The system as claimed in claim 4, wherein the light source (104-1, 104-2, 104,-3, 104-4, 104-5) is configured to emit near infrared light with a wavelength in a range from 750 to 1550 nm.

6. The system as claimed in claim 5, wherein the light source (104-1, 104-2, 104,-3, 104-4, 104-5) is configured to emit near infrared light with a wavelength in a range from 950 to 1000 nm, 1150 to 1250 nm or 1400 to 1500 nm.

7. The system as claimed in any of the preceding claims, wherein the light source comprises a plurality of light emitters (104-1, 104-2, 104,-3, 104-4, 104-5).

8. The system as claimed in claim 7, wherein the light source comprises at least three light emitters (104-1, 104-2, 104,-3, 104-4, 104-5).

9. The system as claimed in claim 7 or 8, wherein the plurality of light emitters (104-1, 104-2, 104,-3, 104-4, 104-5) are configured to sequentially emit light.

10. The system as claimed in any of the preceding claims, wherein the light source (104-1, 104-2, 104,-3, 104-4, 104-5) is configured to emit light with an angle of incidence in a range from 10 to 70 degrees.

11. The system as claimed in claim 10, wherein the light source (104-1, 104-2, 104,-3, 104-4, 104-5) is configured to emit light with an angle of incidence of 22 degrees.

12. A method (600) of operating a system as claimed in any of the preceding claims, the method (600) comprising:
emitting (602) the light (106-1, 106-2) from the light source (104-1, 104-2, 104,-3, 104-4, 104-5);
polarizing (604) the emitted light (106-1, 106-2) in the first polarization direction (110-1, 110-2);
polarizing (606) the light (114-1, 114-2) from the skin (102) in the second polarization direction (116);
detecting (608) the polarized light (114-1, 114-2) from the skin (102);
converting the detected polarized light (114-1, 114-2) from the skin (102) into an image of the skin (102) in a green channel; and
determining the hydration level of the skin (102) based on the detected polarized light (114-1, 114-2) in the image of the skin (102) in a green channel.

13. A computer program product comprising a computer readable medium, the computer readable medium having computer readable code embodied therein, the computer readable code being configured such that, on execution by the processor of the system claimed in any of claims 1 to 11, the processor is caused to operate the system according to the method as claimed in claim 12.

## Patentansprüche

1. System zur Bestimmung eines Feuchtigkeitsniveaus der Haut (102), wobei das System ein Gerät (100) umfasst, wobei das Gerät (100) Folgendes umfasst:
eine Lichtquelle (104-1, 104-2, 104,-3, 104-4, 104-5), die so konfiguriert ist, dass sie Licht (106-1, 106-2) aussendet, um die Haut (102) zu beleuchten;
einen ersten Polarisator (108-1, 108-2), der so angeordnet ist, dass er Licht (106-1, 106-2), das von der Lichtquelle (104-1, 104-2, 104-3, 104-4, 104-5) ausgesendet wird, in einer ersten Polarisationsrichtung (110-1, 110-2, 110-3, 110-4, 110-5) polarisiert, um die Haut (102) mit polarisiertem Licht (106-1, 106-2) zu beleuchten;
einen zweiten Polarisator (112), der so angeordnet ist, dass er Licht (114-1, 114-2) von der Haut (102) in einer zweiten Polarisationsrichtung (116) polarisiert, wobei die zweite Polarisationsrichtung (116) in einem Winkel in einem Bereich ausgerichtet ist von 30 bis 60 Grad in Bezug auf die erste Polarisationsrichtung (110-1, 110-2, 110-3, 110-4, 110-5); und
einen Lichtdetektor (118), der so konfiguriert ist, dass er das polarisierte Licht (114-1, 114-2) von der Haut (102) erkennt;
wobei der Lichtdetektor (118) so konfiguriert ist, dass er das erfasste polarisierte Licht (114-1, 114-2) von der Haut (102) in ein Bild der Haut (102) in einem grünen Kanal umwandelt;
wobei das System einen Prozessor umfasst, der konfiguriert ist für:
Bestimmen eines Hydratationsgrads der Haut (102) basierend auf dem erfassten polarisierten Licht (114-1, 114-2) im Bild der Haut (102) in einem grünen Kanal.

2. System nach Anspruch 1, wobei die zweite Polarisationsrichtung (116) in einem Winkel in einem Bereich von 40 bis 50 Grad in Bezug auf die erste Polarisationsrichtung (110-1, 110-2, 110-3, 110-4, 110-5) ausgerichtet ist.

3. System nach Anspruch 2, wobei die zweite Polarisationsrichtung (116) in einem Winkel von 45 Grad in Bezug auf die erste Polarisationsrichtung (110-1, 110-2, 110-3, 110-4, 110-5).

4. System nach einem der vorhergehenden Ansprüche, wobei die Lichtquelle (104-1, 104-2, 104-3, 104-4, 104-5) so konfiguriert ist, dass sie Licht im nahen Infrarotbereich emittiert.

5. System nach Anspruch 4, wobei die Lichtquelle (104-1, 104-2, 104-3, 104-4, 104-5) so konfiguriert ist, dass sie nahes Infrarotlicht mit einer Wellenlänge in einem Bereich von 750 bis 1550 nm aussendet.

6. System nach Anspruch 5, wobei die Lichtquelle (104-1, 104-2, 104,-3, 104-4, 104-5) so konfiguriert ist, dass sie nahes Infrarotlicht mit einer Wellenlänge in einem Bereich von 950 bis 1000 nm, 1150 bis 1250 nm oder 1400 bis 1500 nm aussendet.

7. System nach einem der vorhergehenden Ansprüche, wobei die Lichtquelle mehrere Lichtsender (104-1, 104-2, 104-3, 104-4, 104-5) umfasst.

8. System nach Anspruch 7, wobei die Lichtquelle mindestens drei Lichtsender (104-1, 104-2, 104-3, 104-4, 104-5) umfasst.

9. System nach Anspruch 7 oder 8, wobei die mehreren Lichtsender (104-1, 104-2, 104-3, 104-4, 104-5) so konfiguriert sind, dass sie nacheinander Licht aussenden.

10. System nach einem der vorhergehenden Ansprüche, wobei die Lichtquelle (104-1, 104-2, 104-3, 104-4, 104-5) so konfiguriert ist, dass sie Licht mit einem Einfallswinkel von a aussendet Bereich von 10 bis 70 Grad.

11. System nach Anspruch 10, wobei die Lichtquelle (104-1, 104-2, 104,-3, 104-4, 104-5) so konfiguriert ist, dass sie Licht mit einem Einfallswinkel von 22 Grad emittiert.

12. Verfahren (600) zum Betreiben eines Systems nach einem der vorhergehenden Ansprüche, wobei das Verfahren (600) Folgendes umfasst:
Emittieren (602) des Lichts (106-1, 106-2) von der Lichtquelle (104-1, 104-2, 104,-3, 104-4, 104-5); Polarisieren (604) des emittierten Lichts (106-1, 106-2) in der ersten Polarisationsrichtung (110-1, 110-2); Polarisieren (606) des Lichts (114-1, 114-2) von der Haut (102) in der zweiten Polarisationsrichtung (116); Erfassen (608) des polarisierten Lichts (114-1, 114-2) von der Haut (102);
Umwandeln der detektierten polarisierten Strahlung (114-1, 114-2) von der Haut (102) in ein Bild der Haut (102) in einem grünen Kanal; und
Bestimmen des Hydratationsgrads der Haut (102) basierend auf der erfassten polarisierten Spannung (114-1, 114-2) im Bild der Haut (102) in einem grünen Kanal.

13. Computerprogrammprodukt, das ein computerlesbares Medium umfasst, wobei das computerlesbare Medium einen darin enthaltenen computerlesbaren Code aufweist, wobei der computerlesbare Code so konfiguriert ist, dass bei Ausführung durch den Prozessor des in einem der Ansprüche 1 bis 11 beanspruchten Systems der Der Prozessor wird veranlasst, das System gemäß dem in Anspruch 12 beanspruchten Verfahren zu betreiben.

## Revendications

1. Système pour déterminer un niveau d'hydratation de la peau (102), dans lequel le système comprend un dispositif (100), le dispositif (100) comprenant:
une source de lumière (104-1, 104-2, 104-3, 104-4, 104-5) configurée pour émettre de la lumière (106-1, 106-2) pour éclairer la peau (102);
un premier polariseur (108-1, 108-2) agencé pour polariser la lumière (106-1, 106-2) émise par la source de lumière (104-1, 104-2, 104,-3, 104-4, 104-5) dans une première direction de polarisation (110-1, 110-2, 110-3, 110-4, 110-5) pour éclairer la peau (102) avec une lumière polarisée (106-1, 106-2);
un second polariseur (112) agencé pour polariser la lumière (114-1, 114-2) provenant de la peau (102) dans une seconde direction de polarisation (116), la seconde direction de polarisation (116) étant orientée selon un angle compris dans une plage de 30 à 60 degrés par rapport à la première direction de polarisation (110-1, 110-2, 110-3, 110-4, 110-5); et
un détecteur de lumière (118) configuré pour détecter la lumière polarisée (114-1, 114-2) provenant de la peau (102);
dans lequel le détecteur de lumière (118) est configuré pour convertir la lumière polarisée détectée (114-1, 114-2) provenant de la peau (102) en une image de la peau (102) dans un canal vert;
dans lequel le système comprend un processeur configuré pour :
déterminer un niveau d'hydratation de la peau (102) sur la base de la lumière polarisée détectée (114-1, 114-2) dans l'image de la peau (102) dans un canal vert.

2. Système selon la revendication 1, dans lequel la deuxième direction de polarisation (116) est orientée selon un angle compris entre 40 et 50 degrés par rapport à la première direction de polarisation (110-1, 110-2, 110-3, 110-4, 110-5).

3. Système selon la revendication 2, dans lequel la deuxième direction de polarisation (116) est orientée selon un angle de 45 degrés par rapport à la première direction de polarisation (110-1, 110-2, 110-3, 110-4, 110-5).

4. Système selon l'une quelconque des revendications précédentes, dans lequel la source de lumière (104-1, 104-2, 104-3, 104-4, 104-5) est configurée pour émettre une lumière proche infrarouge.

5. Système selon la revendication 4, dans lequel la source de lumière (104-1, 104-2, 104-3, 104-4, 104-5) est configurée pour émettre une lumière proche infrarouge avec une longueur d'onde comprise dans une plage de 750 à 1550 nm.

6. Système selon la revendication 5, dans lequel la source de lumière (104-1, 104-2, 104-3, 104-4, 104-5) est configurée pour émettre une lumière proche infrarouge avec une longueur d'onde comprise dans une plage de 950 à 1000 nm, 1150 à 1250 nm ou 1400 à 1500 nm.

7. Système selon l'une quelconque des revendications précédentes, dans lequel la source de lumière comprend une pluralité d'émetteurs de lumière (104-1, 104-2, 104-3, 104-4, 104-5).

8. Système selon la revendication 7, dans lequel la source lumineuse comprend au moins trois émetteurs de lumière (104-1, 104-2, 104-3, 104-4, 104-5).

9. Système selon la revendication 7 ou 8, dans lequel la pluralité d'émetteurs de lumière (104-1, 104-2, 104-3, 104-4, 104-5) est configurée pour émettre séquentiellement de la lumière.

10. Système selon l'une quelconque des revendications précédentes, dans lequel la source de lumière (104-1, 104-2, 104-3, 104-4, 104-5) est configurée pour émettre de la lumière avec un angle d'incidence dans un varie de 10 à 70 degrés.

11. Système selon la revendication 10, dans lequel la source de lumière (104-1, 104-2, 104-3, 104-4, 104-5) est configurée pour émettre de la lumière avec un angle d'incidence de 22 degrés.

12. Procédé (600) d'exploitation d'un système selon l'une quelconque des revendications précédentes, le procédé (600) comprenant:
émettre (602) la lumière (106-1, 106-2) à partir de la source de lumière (104-1, 104-2, 104-3, 104-4, 104-5); polariser (604) la lumière émise (106-1, 106-2) dans la première direction de polarisation (110-1, 110-2); polariser (606) la lumière (114-1, 114-2) provenant de la peau (102) dans la seconde direction de polarisation (116);
détecter (608) la lumière polarisée (114-1, 114-2) provenant de la peau (102);
convertir le film polarisé détecté (114-1, 114-2) provenant de la peau (102) en une image de la peau (102) dans un canal vert; et
déterminer le niveau d'hydratation de la peau (102) sur la base du resserrement polarisé détecté (114-1, 114-2) dans l'image de la peau (102) dans un canal vert.

13. Produit de programme informatique comprenant un support lisible par ordinateur, le support lisible par ordinateur ayant un code lisible par ordinateur incorporé à l'intérieur, le code lisible par ordinateur étant configuré de telle sorte que, lors de l'exécution par le processeur du système revendiqué dans l'une quelconque des revendications 1 à 11, le processeur est amené à faire fonctionner le système selon le procédé tel que revendiqué dans la revendication 12.
